Europäisches Patentamt

(19) European Patent Office

Office européen des brevets

(11) EP 0 568 628 B1

(12) **EUROPEAN PATENT SPECIFICATION**

(45) Date of publication and mention
of the grant of the patent:
**29.09.1999 Bulletin 1999/39**

(21) Application number: **92904962.5**

(22) Date of filing: **21.01.1992**

(51) Int. Cl.$^6$: **A61B 5/00**, G01N 21/31

(86) International application number:
**PCT/US92/00463**

(87) International publication number:
**WO 92/13598** (20.08.1992 Gazette 1992/22)

(54) **TIME AND FREQUENCY DOMAIN SPECTROSCOPY DETERMINING HYPOXIA**

SPEKTROSKOPIE NACH ZEIT- UND FREQUENZ-PARAMETERN ZUR BESTIMMUNG DES SAUERSTOFFMANGELS

SPECTROSCOPIE DANS LE DOMAINE TEMPOREL ET DANS LE DOMAINE FREQUENTIEL DETERMINANT L'HYPOXIE

(84) Designated Contracting States:
**DE FR GB**

(30) Priority: **24.01.1991 US 645590**

(43) Date of publication of application:
**10.11.1993 Bulletin 1993/45**

(73) Proprietor:
**NON-INVASIVE TECHNOLOGY, INC.
Philadelphia, PA 19104 (US)**

(72) Inventor: **CHANCE, Britton
Philadelphia, PA 19103 (US)**

(74) Representative:
**Deans, Michael John Percy et al
Lloyd Wise, Tregear & Co.,
Commonwealth House,
1-19 New Oxford Street
London WC1A 1LW (GB)**

(56) References cited:
US-A- 4 972 331

- **PROCEEDINGS OF THE TWELFTH ANNUAL INTERNATIONAL CONFERENCE OF THE IEEE ENGINEERING IN MEDICINE AND BIOLOGY SOCIETY, vol. 12, 1 - 4 November 1990 PHILADELPHIA (US), pages 2004-2006, XP 000238950 DA BENARON ET AL. 'Non-Invasive Estimation of Cerebral Oxygenation and Oxygen Consumption Using Phase-Shift Spectrophotometry '**
- **ANALYTICAL BIOCHEMISTRY, vol. 195, no. 2, June 1991 pages 330-351, E. M. SEVICK ET AL. 'Quantitation of Time- and Frequency-Resolved Optical Spectra for the Determination of Tissue Oxygenation'**
- **ANALYTICAL BIOCHEMISTRY, vol. 208, no. 2, February 1993 pages 348-351, XP 000561348 M. HAIDA ET AL. 'A Method to Estimate the Ratio of Absorption Coefficients of Two Wavelengths Using Phase-Modulated Near Infrared Light Spectroscopy'**
- **J. Opt. Soc. Am. A, Vol. 4, No. 3, March 1987, R.F. BONNER et al., "Model for photon migration in turbid biological media", pp. 423-432.**

EP 0 568 628 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to spectroscopic examination of tissue.

The present invention arises from our work building upon prior United States Patent Applications Serial No. 287847 filed 21st December 1988 (now US-A-5119815, published after the priority date of the present application), Serial No. 578063 filed 5th September 1990 now US-A-5122974 published after the priority date of the present application), and serial No. 307066 filed 6th February 1989 (now US-A-4972331).

It is clear that a new field of study is emerging where previous limitations to the quantitation of the concentration of absorptive constituents in scattering media by continuous light (CW) approaches are overcome as information on both absorption and scattering parameters become available in homogeneous tissues, and localization possibilities ameliorate problems that arise with inhomogeneous tissues.

The incidence of hypoxia/ischemia and hemorrhage in pre-term neonates is well recognized and the need for early detection of these syndromes is apparent from the current studies using ultrasound and nuclear magnetic resonance. Other applications to neonatology, particularly brain hypoxia monitoring during cardiopulmonary bypass and other surgical procedures applied to the heart and even monitoring of brain hypoxia of the infant in the birth canal as affected by uterine contractions in prolonged deliveries signify other requirements for a reliable method of quantifying oxy-hemoglobin concentrations *in vivo*, in real time. In adults many similar applications emerge, ranging from brain hypoxia in surgical procedures of cardiopulmonary bypass or AICD testing (ventricular defibrillation), including monitoring of oxygenation of recently transplanted, liver, pancreas, etc., to the detection of altered blood flow or lack of blood flow in chronic brain disease such as Alzheimer's, Parkinson's, and multiple infarct dementia (MID). All of these applications dictate an apparatus which can be readily applied to the exposed tissue and collect sufficient data in times as short as the few seconds that may be required to make a significant reading; time is often of the essence in clinical diagnosis and decision making. Thus calibration procedures, etc., must either be made subsequent to the measurement or the system itself should be rapidly auto-calibrating during the study.

A completely different field of applications which also reflect the need for a reliable quantitative measure of oxy- or deoxy-hemoglobin is to the exercising human body either in a confined exercise test such as rowing ergometry, bicycle ergometry, or in strength testing devices, etc., where the motion of the muscle during contraction requires that the unit be firmly attached to the overlying skin. In this case, setup time prior to the exercise should be minimal and recordings of steady state deoxygenation of the muscle bed during exercise and the transient recovery following the exercise is required. Typical applications are to the testing of national rowers, to the triathlon (swimming, running and cycling), or equally important, the training and rehabilitation of muscle function following vascular surgery and the study of muscle atrophy due to extended bed rest, geriatric conditions, or space travel.

Thus, it is clear that a device which makes rapid and reproducible readings of hemoglobin deoxygenation and hemoglobin concentration is highly desirable. To be practical, however, such a device requires a high signal-to-noise ratio and a measurement algorithm that is highly robust, with some possibilities for localization.

The brain cortex and larger muscles of the leg (*Vastus Lateralis*, etc.) are relatively homogeneous whilst the inner layers of the brain and the muscles of the forearm are heterogeneous. Furthermore, diseased tissue, infarcted brain, the necrotic portion of tumors represent heterogeneities that are of especial interest in themselves and indeed if included in the measurement of neighboring tissues would give erroneous values of absorption and scattering. Thus, knowledge of photon propagation in tissues and judicious placement of input/output coupling is required for accurate spectroscopy, or acquisition of data sets appropriate for construction of an image.

In principle, time-resolved spectroscopy converts the measurement of concentration or intensities by transmitted or reflected light to the measurement of photon migration time delay or path length. This enables quantitation of concentration changes in highly scattering tissues which was not heretofore possible.

In U.S. Patent 4 972 331, the present inventor discloses methods and apparatus for studying photon migration using signal modulation techniques such as time, frequency and phase modulation. The photon migration data may be converted, using the principles of time-resolved spectroscopy, to determine the concentration of an absorptive constituent in a scattering medium, such as the concentration of haemoglobin in brain or other tissue of a subject. A practical embodiment of a dual wavelength phase modulation system suitable for clinical application of time-resolved spectroscopy is described. The systems of the said U.S. patent rely upon photon migration between an optical input port and an optical detection port of the spectroscopy system. As photons pass from one to the other through the tissue of the subject, they may be scattered or absorbed.

The phase modulation spectroscopic system described in the aforesaid U.S. Patent 4 972 331 comprises an oscillator which generates a carrier waveform of a selected frequency which is compatible with the time delay of photon migration from the input port to the detection port; a light source which is connected to the oscillator and generates electromagnetic radiation of a selected wavelength that is intensity modulated at the carrier frequency. This radiation is introduced to the subject at the input port. An optical detector detects, at the detection port, the radiation that has migrated in the tissue of the subjects between the input and detection ports.

In accordance with a first aspect of the present invention, we provide a phase modulation spectroscopic system for examination of tissue of a subject, the scattering and absorptive properties of the examined tissue being determined by photons migrating between an optical input port and an optical detection port of said system, said system comprising: an oscillator adapted to generate a carrier waveform of a selected frequency compatible with the time delay of photon migration from said input port to said detection port; a light source, operatively connected to said oscillator, adapted to generate electromagnetic radiation of a selected wavelength that is intensity modulated at said frequency, said radiation being introduced to the subject at said input port; and an optical detector adapted to detect, at said detection port, the radiation that has migrated in said tissue of the subject between said input and detection ports; said system being characterised in further comprising: a phase splitter adapted to produce, based on said introduction radiation, first and second reference phase signals of predefined substantially different phase; first and second double balanced mixers adapted to correlate said reference phase signals and signals of said detected radiation to produce therefrom a real output signal and an imaginary output signal, respectively; and signal processing means adapted to determine, on the basis of said real output signal and said imaginary output signal, a selected characteristic related to the scattering or absorptive properties of said tissue.

According to a second and alternative aspect of the present invention, we provide a method for determining scattering and/or absorptive properties of the tissue of a living subject determined by photons migrating between an optical input port and an optical detection port, the method comprising the steps of: generating a carrier waveform of a selected frequency compatible with the time delay of photon migration from said input port to said detection port; introducing into the subject, at said input port, electromagnetic radiation of a selected wavelength, said radiation having been intensity modulated at said carrier waveform; detecting, at said detection port, the radiation that has migrated in said tissue between said input and detection ports; creating two reference phase signals of predefined substantially different phase values; comparing said detected radiation with said two reference signals and determining therefrom a real output signal and an imaginary output signal, respectively; and, on the basis of said real output signal and said imaginary output signal, calculating a selected characteristic related to the scattering or absorptive properties of the tissue.

We explain below how our spectroscopic methods can provide a system for imaging haemoglobin deoxygenation that fulfills the medical/surgical requirements for patient monitoring and decision making discussed above. However before proceeding to do so, and so as to put the present invention into context we shall describe somewhat related arrangements which, though not in accordance with the present invention enable a better understanding of it.

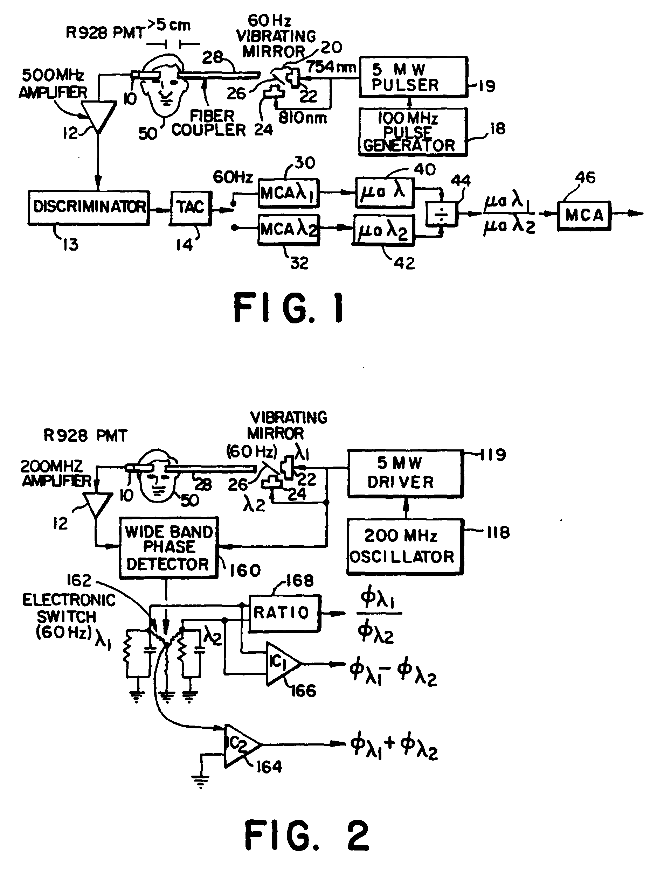

Thus, in one such arrangement not in accordance with this invention described below with reference to Fig. 1, a time sharing, time resolved spectroscopic apparatus for the quantitation of haemoglobin is disclosed which comprises a laser that transmits pulses of light alternately between two wavelengths via a fibre coupler into the subject. A detector receives migrating light and creates a signal which is amplified and then transmitted to a discriminator to provide TTL pulses. The apparatus uses a time to amplitude convertor to create amplitude signals which are then synchronized with the alternations of the frequency and transmitted to two dedicated multichannel analyzers which create an output signal. Finally, a signal processor converts the output signals into a ratio of the terminal slopes of the intensity of the pulses over time and this ratio is directly proportional to the haemoglobin saturation in the tissue of the subject.

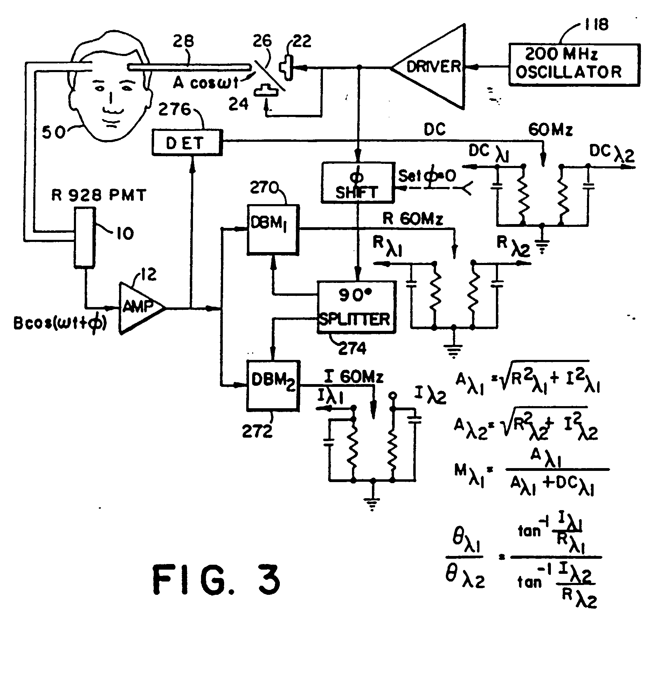

An another arrangement also not in accordance with the present invention, described with reference to Fig. 2, frequency domain spectroscopic apparatus is described which can provide quantitation data for the haemoglobin concentration in the tissue of the subject. In this apparatus, a laser and detector are again used to alternately pulse two wavelengths of electromagnetic radiation into a subject where the received signals are amplified. A phase detector is used to determine the phase shift between the transmitted pulse and the output signal detected. An electronic switch means which is synchronized with the alternations of the pulses separates the alternate wavelengths and transmits them into a signal processor which creates output signals indicative of the sum, the difference and a ratio of the phase signals. These data can be converted into a value of haemoglobin saturation.

In the embodiment of apparatus which is in accordance with the present invention, and which is described with reference to FIG. 3, the spectroscopic apparatus is provided for the quantitation of haemoglobin concentration in a tissue region and again uses an alternately pulsed laser and detector to transmit light through the tissue of a subject. The received signal is again amplified and is then transmitted to two double balanced mixers as well as being transmitted to a phase detector. The double balanced mixers also receive a reference phase shift signal which has been set to zero and fed through a 90° splitter. The output of each of the double balanced mixers is transmitted to sychronizing circuits which respectively separate the real and imaginary portions of the signal for each of the two wavelengths. The phase detector signal is also transmitted to a synchronizing circuit which separates the signal into DC portions corresponding to each of the respective wavelengths. Finally, signal processing means are provided which obtain the process signals indicative of the phase shift and amplitude of the detected signal. These processed signals can then be converted into signals indicative of the modulation index of the tissue at each of the wavelengths.

Methods of determining the concentration of haemoglobin within a tissue region are also disclosed. In the drawings:

FIG. 1 is a schematic representation of a time-resolved spectrophotometer;

FIG. 2 is a schematic representation of a frequency domain spectrophotometer;

neither of the spectrophotometers of FIGS. 1 and 2 being in accordance with the present invention, but both being useful for a better understanding of it; while

FIG. 3 is a schematic representation of an embodiment of time-shared/phase modulated spectrophotometer constructed according to the present invention.

The availability of pulsed laser diode light sources and the knowledge that large tissue volumes such as the adult head exhibit photon migration lasting 5-10 nsec open the possibility that "slow" detection systems such as the squirrel cage photomultipliers, become extremely attractive especially since the latter can be obtained with extended red response which is most suitable for tissue measurements. Silicon diodes, especially of the avalanche type, are of adequate speed but have such a small sensitive area that the need for multiple detectors or appropriate light gathering systems has currently limited their application to large tissue volumes such as adult brain, etc. These components, together with simplified photon counting systems, make time-resolved systems practical for portable use. In fact, currently available tissue spectrophotometers can readily be converted to time domain spectroscopy by the use of pulsed laser diodes and improved photon counting technology.

The configuration of a simplified and portable time domain system for tissue studies is shown in the block diagram of FIG. 1, where a "squirrel cage" photomultiplier detector 10 and a laser diode light source 20 are employed. This system provides a compact time correlated single photon counting (TCSPC) system. As shown in FIG. 1,

Hamamatsu PLP-10 pulsed laser diodes 22, 24 are operated at 10 MHz repetition frequency and at wavelengths of 754 nm and 810 nm. The laser diodes 22, 24 are driven by a 100 MHz pulse generator 18 connected to a 5 mW pulser 19 that drives both diodes. A fiber coupler 28 conducts pulses of light into the subject 50. The light from the two laser diodes 22, 24 is time shared electromechanically by a 60 Hz vibrating mirror 26 so that they alternately illuminate the fiber coupler 28. The transmitted photons migrate through the subject 50 to the detector 10. Using this configuration the extended red sensitive squirrel cage photomultiplier 10 can be employed for studies of human brain at input/output fiber separations of greater than 5 cm as shown. The instrument function shows a 1 nanosecond FWHM response for this detector. The R928 photomultiplier tube 10 for non-imaging spectroscopy can be coupled directly to the forehead to provide a detector area of 200 square millimeters. In an alternate arrangement, a fiber optics coupling (not shown) with an area of 20 square millimeters can be employed --with an obvious decrease in the signal to noise ratio, but providing increased spatial resolution.

The output of the photomultiplier tube 10 is directly connected to a wide band amplifier 12 with appropriate roll-off to give good pulse shape and optimal signal to noise ratio. A high/low level discriminator 13 receives an output signal from the amplifier 12 and gives TTL pulses to a time to amplitude convertor (TAC) 14. Following the time to amplitude conversion, the counts corresponding to the two wavelengths are separately summed in two multichannel analyzers (MCA) 30, 32. The outputs of the multichannel analyzers 30, 32 can be used to calculate signals 40, 41 indicative of the terminal slopes: $\mu_a\lambda_1$ and $\mu_a\lambda_2$. These are divided in a division step 44, since their ratio is simply converted into the saturation of hemoglobin. The pulses are then preferably accumulated in a 1,000 bin multichannel analyzer 46 over a sufficient interval so that approximately $10^5$ counts are collected at the maximum in order that the logarithmic slope be followed down for three or four decades of intensity. The stored information on the slopes of the two wavelengths is then processed by creating a set of ratios and a logarithm is employed in order to conveniently calculate saturation using the formula:

$$Y\,(x\,100\%) = \frac{38-17\dfrac{\mu_a^{754}}{\mu_a^{816}}}{25+9\dfrac{\mu_a^{754}}{\mu_a^{816}}} \qquad (1)$$

This arrangement also permits the scattering factor to be calculated, particularly when fits to the data have been obtained by a diffusion equation. If input/output distances smaller than 5 centimeters ore desired, then the input function can be convoluted with the solution to the diffusion equation, which then is fitted to the experimental curve. Such an instrument has attractive possibilities of portability, and the readout algorithm for saturation is readily computed from the extinction coefficients according to the equation set forth above. This apparatus, with the aid of an extended red sensitive photomultiplier, can accumulate satisfactory data in several minutes, but does not approach the speed of a phase modulated system as explained below.

The data collected and signals obtained by the above apparatus can be further analyzed. The Fourier transform of the domain kinetics give phase and amplitude compounds that contain all the time domain information, and adequate information for concentration determinations in the dual wavelength mode, which is available for both.

Phase modulation spectroscopy has been shown to be well adapted to the measurement of photon migration parameters in the human brain and in model systems. Multi-frequency systems are available and have proved invaluable for studies of the frequency/phase diagrams and for phase modulation imaging of tissues. It has been thought desirable to design simple phase modulation systems for clinical applications. Such designs however, require significant precision, since, as discussed above, it is convenient to employ frequencies within the range of the squirrel cage photomultiplier in systems where the limitations of the photodetector frequencies to 200 MHz, phase shifts of the order a few degrees, and path length changes of a few centimeters are characteristic. Thus, oscillator precision, leakage of excitation signal into the receiving channel, drift of the phase detector, and cross-talk between the channels representing the two wavelengths employed have all been problems.

A simplified system for frequency domain studies is illustrated in FIG. 2. Rather than using a pulse generator, this apparatus utilizes a 200 MHz precision oscillator 118 which drives two laser diodes 22, 24, again at 760 and 816 pm, the outputs of which are time shared into a fiber optic coupling 28 to the head 50 as illustrated. At this frequency, the input/output separations can be varied from 10-5 cm as desired and either the total output from the sensitive area of photocathode (200 square millimeters) can be used or that of a fiber optics coupling from a smaller area, as in the case of the time domain system described above with reference to FIG. 1. Whilst usually an oscillator displaced some 20 KHz from the 200 MHz oscillator is used to provide a low frequency heterodyne signals, the availability of wide band phase detectors makes it attractive to couple the 200 MHz signal directly into such a wide band phase detector chip 160 as indicated in FIG. 2, with time shared outputs corresponding to the two light intensities. In order to demodulate these outputs, an electronic switch 162 synchronized with the vibrating mirror 26 is employed so that the phase delay at the two wavelengths is available as the ratio the difference, or the sum for appropriate calculations of the saturation according to the equality:

$$\frac{\theta^{\lambda_1} - \theta_0^{\lambda_1}}{\theta^{\lambda_2} - \theta_0^{\lambda_2}} = \frac{\mu_a^{\lambda_1}}{\mu_a^{\lambda_2}}$$

Research has shown that $\theta_0$ can be determined from the hemoglobin free scattering properties of the brain tissue. Thus, the three outputs of the electronic switch 162 may be fed to processing circuits 164, 166 which create a sum and difference respectively. The phase outputs are also combined in a circuit 168 to create a ratio. The selection and construction of the logic devices, e.g., the integrated circuits disclosed herein and the like, is well known to those of ordinary skill.

The term $\theta_0$ limits the applications of single-frequency modulated spectroscopy to tissues where $(1-g)\mu_s$ can be estimated a priori and is not expected to change such as might happen during disease, treatments such as radiotherapy, or even transfer from animal models to tissue. There are, however, two approaches to utilizing the approximation which circumvent this problem: (i) to employ an additional third wavelength; and (ii) to employ dual-wavelength, dual-frequency phase-modulation techniques. In the first approach, the ratio of absorption coefficients for two sets of wavelengths are used to solve for $\theta_0$ such that both ratios of absorption coefficients predict identical hemoglobin saturations, Y. In the second approach, measurement of phase shifts at dual-wavelength and dual-frequencies, where $2\pi f_1$, $2\pi f_2 >> \mu_a^{\lambda_1} c$, $\mu_a^{\lambda_2} c$, can give information of hemoglobin saturation from transmittance geometries.

In the arrangement of FIG. 2, a block diagram of a time shared phase modulation system in which two wavelengths are available is shown. This system provides appropriate sums and differences and ratios of the signal utilizing a vibrating mirror 26 for time sharing of the two laser wavelengths. This simplifies the oscillator system, as only two are required and only one phase detector. The difference or ratio circuits afford cancellation of common mode errors to a great extent affording high performance with a simple system.

An alternate way of handling the data output from the system of FIG. 2 is illustrated in FIG. 3, an arrangement constructed in accordance with the present invention, where the detector output is put through two wide band double balance mixers (DBM) 270, 272 which are fed through a 90° phase splitter 274 so that real (R) and imaginary (I) portions of the signal are obtained. The double balance mixers 270, 272 preferably operate at the modulation frequency. The phase $\theta$ is the angle whose tangent is the imaginary over real part, whilst the amplitude is the square root of the sum of the squares of these values- providing the phase shift has been taken out as the residual phase shift $\theta$ set to zero as indicated. Thus this embodiment of the present invention provides the modulation index, which is the quotient of the amplitude over the amplitude plus the DC component obtained from a narrow band detector 276 as shown in FIG. 3. A synchronous detector decodes the phase shifts for the phase and amplitude values for the two wavelengths so that the ratio of the phase shifts may be obtained as indicated in the previous diagrams. In order to obtain the proper functions, the ratio of the $\theta$'s and the correct value of amplitude, a ratio circuit divides the I and R terms and the angle is computed; in this case, the small angle approximation is valid and the ratio circuit computes $\theta\lambda_1/\theta\lambda_2$ as required for the equation set forth with reference to Fig. 2 above. In the case of the amplitude function, the square root of the sum of the squares are computed to obtain the amplitude and the summing and dividing circuits calculate the modulation index at the two wavelengths.

Some of the components of the apparatus described above with reference to Figs. 1-3 are of especial interest, e.g., the requirements for the laser diodes 22, 24 are that the low power, 5 mW laser diodes be modulated near 100% to give signals appropriate for tissue studies. The phase modulated information is obtained from a significant volume of tissue surface. A second and important feature is the stability of crystal oscillators requiring appropriate electronic construction and tuning parameters. The performance values are drift of 0.2mm/hr and phase noise < 0.9mm in a 0.1 Hz band width. Signal changes are usually 1-2 cm in a 23 cm path length. Wavelengths of the laser diodes 22, 24 are preferably on opposite sides of the cross over or isosbestic point between the oxy- and deoxy- hemoglobin absorption spectrum (about 800 nm) are used so that the difference of the signals represents a change on deoxygenation whilst the sum represents the total amount of hemoglobin present of the desirable quantities mentioned above.

Data display is of considerable importance to the functioning of the apparatus, as is computer coupling. A running time LCD display is preferably used for monitoring in order to ensure that signals are within the linear range of the phase detector, together with a LED indicator, indicating that the signal amplitude does not reach predetermined limits. In addition, the computer coupling to obtain manipulated data from wave saturation may be obtained as necessary.

Various formulations by which the path length changes measured in time and frequency domain studies can be simply converted into concentration changes using appropriate wavelengths and appropriate extinction coefficients have been presented. Whilst the biochemist requires the determination of a tissue concentration, the physiologist is content with the saturation percentage change of one form with respect to another, usually the oxy with respect to the total (oxy + deoxy) in the case of hemoglobin. These algorithms generally involve the ratio of the path lengths determined at a pair of wavelengths as indicated in the discussion above. However, those of ordinary skill will appreciate that other absorbers can also be studied. For example, injected indocyanine-green (a flow indicator), or naturally occurring absorbers such as fat, protein, water may be studied by photon migration techniques such as those described above.

The coupling of the system to the subject 50 is of importance and whilst this can be done satisfactorily with plastic light guides 28, usually of an area for receiving of 2 square centimeters, direct contact of a large area detector with the subject's skin is desirable if the input/output separation exceeds 5-10 cm, as is the case in an adult. Similarly, whilst spectroscopy requires signal acquisition from large surface areas, imaging upon this area sets limitations to about 1 cm signals from a large number of points around the circumference of the human head are desirable for planar imaging of brain bleeding.

The performance of the phase modulation systems disclosed above have been experimentally determined, together with quantification of the drift, noise and other parameters. For example, the performance of the laser diode light source and squirrel cage detector as applied to a model system and a human head have been noted. While animal models provide excellent systems for quantifying the performance of both time resolved and phase modulated systems the exercising human muscle is optimal for the validation of the functionality of the system, particularly in terms of the signal-to-noise ratio in an actual *in vivo* system.

It will thus be seen that we have described herein three systems for time and frequency domain spectroscopy, each one capable of measurement of haemoglobin saturation and blood volume, but, of which, only the FIG. 3 arrangement is in accordance with the present invention. In the apparatus of Figure 1 the entire time profile of photon decay is measured and if measured at longer times, is independent of $\mu_s$. Perhaps the most important feature of the instrumentation is that the variation of time delay with photomultiplier voltage is not as a primary factor, as long as the logarithmic slopes, $\mu_a$ values, are read out at longer times, i.e., 5-10 ns. Thus, this system is optimal for the adult human head where long path lengths and large separations of input/output are available.

The systems of Figures 2 and 3 are suitable for shorter path lengths as are observed in skeletal muscle or neonates. In particular the system of Figure 3 in accordance with this invention can give comprehensive information which if available at a number of frequencies and appropriately Fourier transformed would have the same information content as the pulse time method. When restricted to particular carrier frequencies, the method nevertheless retains its quantitation of hemoglobin saturation through the ratio of the $\theta$ values and affords in addition the modulation index. Thus, this unit would have unique properties for imaging- brain bleeding, or other localized depots of hemoglobin. The system is in effect significantly faster than that of Figure 1 by a factor of 10 or perhaps more.

The system of Figure 2 in the simplest system requiring only a small number of chips to afford the ratio of phase shifts necessary for the calculation of saturation. This system is also quite fast, time constants of 5 sec probably being appropriate for brain recording.

The system of Figure 1 is not expected to need calibration except for its own instrument function which is expected to be constant for a given diode voltage on the photomultiplier and to vary insignificantly with small changes thereof. The systems of Figures 2 and 3 require calibration for the values of θ although it should be noted that the ratio of these values is acceptable and thus the calibration errors will only be of secondary importance. Obviously, the rapid development of available electronic circuitry, particularly in the region of 100-500 MHz will result in further simplifications of the apparatus displayed here. At the present time, the use of time shared laser diode wavelengths and the calculation from the ratio of phase shifts of the saturation value allows much greater freedom from drift and background signals than for a single wavelength system. Obviously, as many wavelengths as desired can be obtained by time sharing.

To acquire appropriate data for the study of brain tissue hypoxia using apparatus described herein, the first requirement is obviously adequate accuracy and reproducibility; the requirements being approximately 1 mm in distance or 0.001 in logarithmic slope (i.e., a corresponding value in mm). Absolute stability is less stringent, but nevertheless, the measurement requires the difference or ratio of slopes for the determination of saturation and for the absolute concentration. The second major requirement is to select wavelengths at which an adequate signal is generated, i.e., one in which the change of hemoglobin saturation or concentration gives a significant change of path length. Also, ready calibration is necessary. For animal models, 100% change can readily be obtained by ischemia and hypoxia, however, for the study of human subjects, the range from 40% to 80% saturation is the maximum that could be expected under conditions of patient stability. The "normal" variations may be 1/5th of this for 8 to 10%. Thus, the "oxygen saturation" of the brain study requires a very high level of stability and reproducibility and stable calibration.

As explained above, we can determine hemoglobin saturation from measurements of phase-shift and modulation at varying modulation frequency. It has been found that $\mu_a^\lambda$ may be identified from reflectance and transmittance measurements of phase-shift, θ, and demodulation of the detected signal, M, as a function of modulation frequency, f. Theoretical considerations show that the critical phase and modulation frequencies, $f_o'$ and $f_o^M$, at which the magnitude of the θ and M versus f slopes reach minima, are functions of $\mu_a$ alone, regardless of $(1-g)\mu_s$ or of the source and detector configuration. From the ratio of critical frequencies identified from phase-shift and modulation spectra, the ratio of absorption coefficients can be found:

$$\frac{f_o^{\mu,\lambda_1}}{f_o^{\mu,\lambda_2}} = \frac{f_o^{\theta,\lambda_1}}{f_o^{\theta,\lambda_2}} = \frac{\mu_a^{\lambda_1}}{\mu_a^{\lambda_2}}$$

Sensitivity of this technique increases with enhanced tissue scattering and minimal absorption as is the characteristic of the brain. Thus, $f_o'$ and $f_o^M$, might be identified from experimental spectra of media with physiological scattering properties to determine whether differential frequency-resolved spectroscopy may be used to accurately quantitate tissue oxygenation.

The invention may be utilised to provide a spectroscopic method of examination of tissue of a subject, the subject lying between a optical input port and an optical detection port of a phase modulation spectroscopic system, the optical pathlength of photons migrating between said ports being determined by the scattering and absorptive properties of the examined tissue, the method comprising the steps of:

generating a easier waveform of a selected frequency compatible with the time delay of photon migration from said input port to said detection port, introducing into the subject, at said input port, electomagnetic radiation of a selected wavelength, said radiation having been intensity modulated at said carrier waveform;
detecting, at said detection port, the radiation that has migrated in said tissue between said input and detection ports;
creating two reference phase signals of predefined phase value that differ by 90 degrees;
comparing said detected radiation with said two reference signals and determining therefrom a real output signal and a imaginary output signal, respectively; and
on the basis of said real output signal and said imaginary output signal, by calculating a selected characteristic related to the scattering or absorptive properties of the tissue.

Such a method may further comprise the steps of:

introducing into the subject at said input port electromagnetic radiation of a second selected wavelength modulated by said carrier waveform;
detecting, at said detection port, the radiation of said second wavelength that has migrated in said tissue between said input and detection ports;
comparing said detected radiation of said second wavelength with said two reference signals and determining therefrom a real output signal at said second wavelength and an imaginary output signal at said second wavelength, respectively; and
on the basis of said real and imaginary output signal at each of said selected wavelengths, the tissue by calculating a selected characteristic related to the scattering or absorptive properties of the tissue.

Further steps may include introducing into the subject at said input port electromagnetic radiation of a third selected wavelength modulated at said carrier waveform;

detecting, at said detection port, the radiation of said third wavelength that has migrated in said tissue between said input and detection ports;
comparing said detected radiation of said third wavelength with said two reference signals and determining therefrom a real output signal at said third wavelength and an imaginary output signal at said third wavelength, respectively.

In a method of this type said steps may be formed at a second selected frequency and wherein said tissue is examined by employing selected characteristics determined at at least two wavelengths and at least two frequencies. Said selected wavelengths may be in the range of visible and infra red wavelengths. The selected characteristic referred to above may be a signal amplitude determined as the square root of the sum of the squares of said real output signal and said imaginary output signal. In this last case the said method may further comprise the steps of:

determining DC signal from said detected radiation; and
calculating the modulation index based on said DC signal and said signal amplitude.

The selected characteristic may also be a phase shift, determined at at least one of said selected wavelengths, between the radiation introduced at said input port and the radiation detected at said detection port. In which case the following further steps may be taken; determining the ratio of absorption coefficients of said radiation at each said wavelength based on the ratio of the correponding phase shifts; or further determining the saturation of haemoglobin of said examined tissue based on said ratio of absorption coefficients, or determining localized bleeding in said tissue.

The selected characteristic may also be the photon migration pathlength, determined at at least one of said selected wavelengths, between the radiation introduced at said input port and the radiation detected at said detection port.

Said tissue may be imaged by employing at least one of said selected characteristics at said wavelengths.

**Claims**

1. A phase modulation spectroscopic system for examination of tissue of a subject, the scattering and absorptive properties of the examined tissue being determined by photons migrating between an optical input port and an optical detection port of said system, said system comprising: an oscillator adapted to generate a carrier waveform of a selected frequency compatible with the time delay of photon migration from said input port to said detection port; a light source, operatively connected to said oscillator, adapted to generate electromagnetic radiation of a selected wavelength that is intensity modulated at said frequency, said radiation being introduced to the subject at said input port; and an optical detector adapted to detect, at said detection port, the radiation that has migrated in said tissue of the subject between said input and detection ports; said system being characterised in further comprising: a phase splitter adapted to produce, based on said introduction radiation, first and second reference phase signals of predefined substantially different phase; first and second double balanced mixers adapted to correlate said reference phase signals and signals of said detected radiation to produce therefrom a real output signal and an imaginary output signal, respectively; and signal processing means adapted to determine, on the basis of said real output signal and said imaginary output signal, a selected characteristic related to the scattering or absorptive properties of said tissue.

2. A spectroscopic system according to Claim 1, characterised in further comprising: a light source, operatively connected to said oscillator, adapted to generate electromagnetic radiation of a second selected wavelength that is intensity modulated at said frequency; a coupler adapted to introduce radiation at each said wavelength to said subject at said input port; said optical detector being further adapted to detect, at said detection port, the radiation of said second wavelength that has migrated in said tissue between said input and detection ports; and a first and a second electronic switch, both operating synchronized with said coupler, arranged to receive, respectively, said real output signal and said imaginary output signal from said first and said second double balanced mixers, said switches being adapted to separate said real and imaginary signals to signals, respectively, at each of said wavelengths.

3. A spectroscopic system according to Claim 2, characterised in further comprising: a light source, operatively connected to said oscillator, adapted to generate electromagnetic radiation of a third selected wavelength that is intensity modulated at said frequency; said optical detector being further adapted to detect, at said detection port, the radiation of said third wavelength that has migrated in said tissue between said input and detection ports; and said first and said second electronic switches, both operating synchronized with said coupler, being further arranged to receive said real output signal and said imaginary output signal from said first and said second double balanced mixers, respectively, and being further adapted to separate said real and imaginary signals, respectively, at each of the three wavelengths.

4. A spectroscopic system according to any of Claims 1, 2 or 3, further characterised in that said selected characteristic is a signal amplitude, at at least one selected wavelength, determined as the square root of the sum of the squares of said real output signal and said imaginary output signal at said wavelength.

5. A spectroscopic system according to any of Claims 2, 3 or 4, characterised in further comprising: a narrow band detector adapted to receive detected signals from said optical detector and to produce a DC output signal therefrom; a third electronic switch operating synchronized with said coupler, arranged to receive said DC output signal from said narrow band detector and adapted to separate said DC signal at each of said wavelengths; and said signal processing means being further adapted to determine a modulation index, at at least one of said wavelengths, as the ratio of values of said signal amplitude and said signal amplitude plus said DC output signal.

6. A spectroscopic system according to any of Claims 1, 2 or 3, further characterised in that said selected characteristic is phase shift between the radiation introduced at said input port and the radiation detected at said detection port, determined at at least one of said selected wavelengths.

7. A spectroscopic system according to Claim 6, further characterised in that said signal processing means is further adapted to determine the ratio of said phase shifts at two selected wavelengths.

8. A spectroscopic system according to Claim 7, further characterised in that said signal processing means is further adapted to determine the saturation of haemoglobin based on said ratio of phase shifts.

9. A spectroscopic system according to any of Claims 1, 2 or 3, further characterised in that said selected characteristic is the migration pathlength between said input and detection ports of radiation at at least one of said selected wavelengths.

10. A spectroscopic system according to any of Claims 1, 2 or 3, further characterised in that said oscillator is further adapted to operate at a different selected frequency of said carrier waveform.

11. A spectroscopic system according to Claim 10, further characterised in that said selected characteristic of said tissue is a phase shift between the radiation introduced at said input port and the radiation detected at said detection port determined at at least two wavelengths and at least two selected frequencies; and in that said signal processing means is further adapted to determine saturation of haemoglobin based on said phase shifts.

12. A spectroscopic system according to any one of the above claims, further characterised in that said wavelengths are in the range of visible and infrared wavelengths.

13. A method for determining scattering and/or absorptive properties of the tissue of a living subject determined by photons migrating between an optical input port and an optical detection port, the method comprising the steps of: generating a carrier waveform of a selected frequency compatible with the time delay of photon migration from said input port to said detection port; introducing into the subject, at said input port, electromagnetic radiation of a selected wavelength, said radiation having been intensity modulated at said carrier waveform; detecting, at said detection port, the radiation that has migrated in said tissue between said input and detection ports; creating two reference phase signals of predefined substantially different phase values; comparing said detected radiation with said two reference signals and determining therefrom a real output signal and an imaginary output signal, respectively; and, on the basis of said real output signal and said imaginary output signal, calculating a selected characteristic related to the scattering or absorptive properties of the tissue.

14. A method according to Claim 13, further comprising the steps of: introducing into the subject at said input port electromagnetic radiation of a second selected wavelength modulated by said carrier waveform; detecting, at said detection port, the radiation of said second wavelength that has migrated in said tissue between said input and detection ports; comparing said detected radiation of said second wavelength with said two reference signals and determining therefrom a real output signal at said second wavelength and an imaginary output signal at said second wavelength, respectively; and on the basis of said real and imaginary output signal at each of said selected wavelengths, calculating a selected characteristic related to the scattering or absorptive properties of the tissue.

15. A method according to Claim 14, further comprising the steps of: introducing into the subject at said input port electromagnetic radiation of a third selected wavelength modulated at said carrier waveform; detecting, at said detection port, the radiation of said third wavelength that has migrated in said tissue between said input and detection ports; comparing said detected radiation of said third wavelength with said two reference signals and determining thereform a real output signal at said third wavelength and an imaginary output signal at said third wavelength, respectively.

16. A method according to any of Claims 13, 14 and 15 repeated at a second selected frequency, said scattering and absorptive properties of said tissue being examined by employing selected characteristics determined at at least two wavelengths and at least two frequencies.

17. A method according to any of Claims 14, 15 or 16 wherein said selected wavelengths are in the range of visible and infrared wavelengths.

18. A method according to any of Claims 13 to 17, wherein the selected characteristic is a signal amplitude determined as the square root of the sum of the squares of said real output signal and said imaginary output signal.

19. A method according to Claim 18, wherein said method further comprises the steps of: determining DC signal from said detected radiation; and calculating the modulation index based on said DC signal and said signal amplitude.

20. A method according to any of Claims 14 to 17, wherein the selected characteristic is a phase shift, determined at at least one of said selected wavelengths, between the radiation introduced at said input port and the radiation detected at said detection port.

21. A method according to Claim 20, further comprising the steps of: determining the ratio of absorption coefficients of said radiation at each said wavelength based on the ratio of the corresponding phase shifts.

22. A method for determining the saturation of haemoglobin of said examined tissue or of providing a numerical value useful in determination of localized bleeding in said tissue comprising determining said ratio of absorption coefficients in a method according to Claim 21.

23. A method according to any of Claims 14 to 17, wherein the selected characteristic is the photon migration pathlength, determined at at least one of said selected wavelengths, between the radiation introduced at said input port and the radiation detected at said detection port.

**Patentansprüche**

1. Phasenmodulations-Spektroskopsystem zur Untersuchung des Gewebes eines Subjektes, wobei die Streu- und Absorptionseigenschaften des untersuchten Gewebes durch Photonen bestimmt werden, die zwischen einem optischen Eingangsanschluß und einem optischen Detektionsanschluß des Systems wandern, wobei das System folgendes aufweist:

einen Oszillator, der geeignet ist eine Trägerwellenform einer ausgewählten Frequenz zu erzeugen, und zwar kompatibel mit der Zeitverzögerung der Photonenwanderung von dem Eingangsanschluß zu dem Detektionsanschluß,
eine Lichtquelle, die betriebsmäßig mit dem Oszillator verbunden und geeignet ist, elektromagnetische Strahlung einer ausgewählten Wellenlänge zu erzeugen, die bei der erwähnten Frequenz intensitätsmoduliert ist, wobei die Strahlung in das Subjekt an dem Eingangsanschluß eingeführt wird; und
einen optischen Detektor geeignet zur Detektion an dem Detektionsanschluß der Strahlung, die in dem erwähnten Gewebe des Subjektes zwischen dem Eingangs- und Detektionsanschlüssen gewandert ist; wobei das System dadurch gekennzeichnet ist, daß es ferner folgendes aufweist:
einen Phasenteiler geeignet zur Erzeugung basierend auf der erwähnten eingeführten Strahlung, erste und zweite Bezugsphasensignale von vordefinierter im wesentlichen unterschiedlicher Phase;
erste und zweite doppelt ausgeglichene Mischer, geeignet um die erwähnten Bezugsphasensignale und die Signale der erwähnten detektierten Strahlung in Korrelation zu bringen zur Erzeugung eines realen Ausgangssignals bzw. eines imaginären Ausgangssignals daraus; und
Signalverarbeitungsmittel, geeignet zur Bestimmung auf der Basis des erwähnten realen Ausgangssignals und des erwähnten imaginären Ausgangssignals einer ausgewählten Charakteristik die mit den Streu- oder Absorptionseigenschaften des erwähnten Gewebes in Beziehung steht.

2. Spektroskopsystem nach Anspruch 1, dadurch gekennzeichnet, daß ferner folgendes vorgesehen ist:

eine betriebsmäßig mit dem Oszillator verbundenen Lichtquelle geeignet zur Erzeugung elektromagnetischer Strahlung einer zweiten ausgewählten Wellenlänge die mit der erwähnten Frequenz intensitätsmoduliert ist;
ein Koppler, geeignet zur Einführung von Strahlung mit jeder der erwähnten Wellenlänge zum Subjekt an dem erwähnten Eingangsanschluß;
wobei der erwähnte optische Detektor ferner geeignet ist an dem erwähnten Detektionsanschluß die Strahlung der erwähnten zweiten Wellenlänge zu detektieren, die in dem erwähnten Gewebe zwischen den Eingangs- und Detektionsanschlüssen gewandert ist; und einen ersten und zweiten elektronischen Schalter die beide synchron mit dem Koppler arbeiten, um jeweils das erwähnte reale Ausgangssignal bzw. das erwähnte imaginäre Ausgangssignal von den ersten und den zweiten doppelt ausgeglichenen Mischern zu empfangen, wobei die Schalter geeignet sind die realen und imaginären Signale zu trennen, und zwar jeweils in Signale auf jeder der enwähnten Wellenlängen.

3. Spektroskopsystem nach Anspruch 2, dadurch gekennzeichnet, daß ferner folgendes vorgesehen ist:

eine betriebsmäßig mit dem Oszillator verbundenen Lichtquelle geeignet zur Erzeugung elektromagnetischer Strahlung einer dritten ausgewählten Wellenlänge die mit der erwähnten Frequenz intensitätsmoduliert ist;
wobei der optische Detektor ferner geeignet ist, um an dem Detektionsanschluß die Strahlung der erwähnten dritten Wellenlänge zu detektieren, die in dem Gewebe zwischen den Eingangs- und Detektionsanschlüssen gewandert ist; und wobei die ersten und zweiten elektronischen Schalter die beide synchron mit dem Koppler arbeiten ferner derart angeordnet sind, daß sie das erwähnte reale Ausgangssignal und das erwähnte imaginäre Ausgangssignal von dem ersten bzw. zweiten doppelt ausgeglichenen Mischer zu empfangen, und ferner dazu geeignet sind, um die realen bzw. imaginären Signale an jeder der drei Wellenlängen zu trennen.

4. Spektroskopsystem nach irgendeinem der Ansprüche 1, 2 oder 3, dadurch gekennzeichnet, daß die ausgewählte Charakteristik eine Signalamplitude ist, bei mindestens einer ausgewählten Wellenlänge bestimmt als die Quadratwurzel der Summe der Quadrate des realen Ausgangssignals und des erwähnten imaginären Ausgangssignals auf der Wellenlänge.

5. Spektroskopsystem nach einem der Ansprüche 2, 3 oder 4 ferner dadurch gekennzeichnet, daß folgendes vorgesehen ist;

ein Schmalbanddetektor, geeignet zum Empfang von detektierten Signalen von dem optischen Detektor und zur Erzeugung eines Gleichstromausgangssignals daraus;
ein dritter elektronischer Schalter, der synchron mit dem Koppler arbeitet und derart angeordnet ist, daß er das Gleichstromausgangssignal von dem erwähnten Schmalbanddetektor empfängt und geeignet ist, um das Gleichstromsignal bei jeder der erwähnten Wellenlängen zu trennen; und wobei die Signalverarbeitungsmittel ferner geeignet sind, um einen Modulationsindex zu bestimmen, und zwar bei mindestens einer der erwähnten Wellenlängen als das Verhältnis von Werten der erwähnten Signalamplitude und der erwähnten Signalamplitude plus dem Gleichstromausgangssignal.

6. Spektroskopsystem nach einem der Ansprüche 1, 2 oder 3, ferner dadurch gekennzeichnet, daß die erwähnte ausgewählte Charakteristik eine Phasenverschiebung zwischen der Strahlung eingeführt an dem Eingangsanschluß und der Strahlung detektiert am Detektionsanschluß ist, und zwar bestimmt bei mindestens einer der erwähnten ausgewählten Wellenlängen.

7. Spektroskopsystem nach Anspruch 6, ferner dadurch gekennzeichnet, daß die Signalverarbeitungsmittel ferner geeignet sind, um das Verhältnis der erwähnten Phasenverschiebrungen bei zwei ausgewählten Wellenlängen zu bestimmen.

8. Spektroskopsystem nach Anspruch 7, ferner dadurch gekennzeichnet, daß die Signalverarbeitungsmittel ferner geeignet sind, um die Sättigung an Hämoglobin zu bestimmen, und zwar basierend auf dem erwähnten Verhältnis der Phasenverschiebungen.

9. Spektroskopsystem nach einem der Ansprüche 1, 2 oder 3, ferner dadurch gekennzeichnet, daß die ausgewählte Charakteristik die Wanderungspfadlänge zwischen den Eingangs- und Detektionsanschlüssen der Strahlung auf mindestens eine der ausgewählten Wellenlängen ist.

10. Spektroskopsystem nach einem der Ansprüche 1, 2 oder 3, ferner dadurch gekennzeichnet, daß der Oszillator ferner geeignet ist, um auf einer unterschiedlich ausgelegten Frequenz der Trägerwellenform zu arbeiten.

11. Spektroskopsystem nach Anspruch 10, dadurch gekennzeichnet, daß die ausgewählte Charakteristik des Gewebes eine Phasenverschiebung ist, und zwar zwischen der an dem Eingangsanschluß eingeführten Strahlung und der an dem Detektionsanschluß detektierten Strahlung, und zwar bestimmt bei mindestens zwei Wellenlängen und bei mindestens zwei ausgewählten Frequenzen, und ferner dadurch gekennzeichnet, daß die Signalverarbeitungsmittel ferner dazu geeignet sind, um die Sättigung des Hämoglobins zu bestimmen, und zwar basierend auf den erwähnten Phasenverschiebungen.

12. Spektroskopsystem nach einem der vorstehenden Ansprüche, ferner dadurch gekennzeichnet, daß die erwähnten Wellenlängen in dem Bereich sichtbarer und infraroter Wellenlängen liegen.

13. Verfahren zur Bestimmung von Streu- und/oder Absorptionseigenschaften des Gewebes eines lebenden Subjektes, bestimmt durch die zwischen einem optischen Eingangsanschluß und einem optischen Detektionsanschluß wandernden Photonen, wobei das Verfahren die folgenden Schritte vorsieht:

Erzeugen einer Trägerwellenform mit einer ausgewählten Frequenz kompatibel mit der Zeitverzögerung der Photonenwanderung von dem erwähnten Eingangsanschluß zu dem Detektionsanschluß;
Einführung elektromagnetischer Strahlung mit einer ausgewählten Wellenlänge an dem Eingangsanschluß in das Subjekt, wobei die Strahlung bei der Trägerwellenform intensitätsmoduliert ist;
Detektieren am Detektionsanschluß der Strahlung, die in dem Gewebe zwischen den Eingangs- und Detektionsanschlüssen gewandert ist;
Erzeugen von zwei Referenzphasensignalen mit vordefinierten im wesentlichen unterschiedlichen Phasenwerten;
Vergleichen der detektierten Strahlung mit den zwei Bezugs- oder Referenzsignalen und Bestimmung eines realen Ausgangssignals bzw. eines imaginären Ausgangssignals daraus; und

Berechnen auf der Basis des realen Ausgangssignals und des imaginären Ausgangssignals, einer ausgewählte Charakteristik, die mit den Streu- oder

Absorptionseigenschaften des Gewebes in Beziehung steht.

14. Verfahren nach Anspruch 13, wobei ferner die folgenden Schritte vorgesehen sind:

Einführen elektromagnetischer Strahlung mit einer ausgewählten zweiten Wellenlänge moduliert bei der Trägerwellenform in das Subjekt in dem Eingangsanschluß;
Detektieren am Detektionsanschluß der Strahlung der zweiten Wellenlänge, die in dem Gewebe zwischen den Eingangs- und Deteketionsanschlüssen gewandert ist;
Vergleichen der detektierten Strahlung der erwähnten zweiten Wellenlänge mit den erwähnten zwei Bezugssignalen und Bestimmung eines realen Ausgangssignals bei der erwähnten zweiten Wellenlänge bzw. eines imaginären Ausgangssignals bei der zweiten Wellenlange daraus; und
Berechnen auf der Basis des erwähnten realen und imaginären Ausgangssignals bei jeder der erwähnten ausgewählten Wellenlängen eine ausgewählte Charakteristik, die mit den Streu- oder Absorptionseigenschaften des Gewebes in Beziehung steht.

15. Verfahren nach Anspruch 14, wobei folgende Schritte vorgesehen sind:

Einführen elektromagnetischer Strahlung einer dritten ausgewählten Wellenlänge moduliert bei der Trägerwellenform in das Subjekt an dem Eingangsanschluß;
Detektieren am Detektionsanschluß der Strahlung der erwähnten dritten Wellenlänge, die in dem Gewebe zwischen den Eingangs- und Deteketionsanschlüssen gewandert ist;
Vergleichen der erwähnten detektierten Strahlung der erwähnten dritten Wellenlänge mit den zwei Bezugssignalen und Bestimmung eines realen Ausgangssignals bei der dritten Wellenlänge bzw. eines imaginären Ausgangssignals bei der dritten Wellenlänge daraus.

16. Verfahren nach irgendeinem der Ansprüche 13, 14 und 15, und zwar wiederholt bei der zweiten ausgewählten Frequenz, wobei die Streu- und Absorptionseigenschaften des Gewebes, dadurch untersucht werden, daß man ausgewählte Charakteristika bestimmt bei mindestens zwei Wellenlänge und bei mindestens zwei Frequenzen verwendet.

17. Verfahren nach einem der Ansprüche 14, 15 oder 16, wobei die ausgewählten Wellenlängen im Bereich sichtbarer und infraroter Wellenlängen liegen.

18. Verfahren nach einem der Ansprüche 13 bis 17, wobei die ausgewählte Charakteristik eine Signalamplitude ist, und zwar bestimmt als die Quadratwurzel der Summe der Quadrate des realen Ausgangssignals und des imaginären Ausgangssignals.

19. Verfahren nach Anspruch 18, wobei das Verfahren ferner die folgenden Schritte aufweist:

Bestimmen eines Gleichstromsignals aus der detektierten Strahlung und Berechnen des Modulationsindex basierend auf dem Gleichstromsignal und der erwähnten Signalamplitude.

20. Verfahren nach einem der Ansprüche 14 bis 17, wobei die ausgewählte Charakteristik eine Phasenverschiebung ist, und zwar bestimmt bei mindestens einer der erwähnten ausgewählten Wellenlängen zwischen der Strahlung eingeführt an dem Eingangsanschluß und der Strahlung detektiert an dem Detektionsanschluß.

21. Verfahren nach Anspruch 20, wobei ferner die folgenden Schritte vorgesehen sind:

Bestimmen des Verhältnisses von Absorptionskoeffizienten der Strahlung bei jeder der Wellenlängen basierend auf dem Verhältnis der entsprechenden Phasenverschiebungen.

22. Verfahren zur Bestimmung der Sättigung des Hämoglobins des untersuchten Gewebes oder Vorsehen eines numerischen Werts brauchbar bei der Bestimmung einer örtlichen Blutung in dem Gewebe, wobei die Bestimmung des Verhältnisses der Absorptionskoeffizienten gemäß einem Verfahren nach Anspruch 21 erfolgt.

23. Verfahren nach einem der Ansprüche 14 bis 17, wobei die ausgewählte Charakteristik die Photonenwanderungspfadlänge ist, und zwar bestimmt bei mindestens einer der ausgewählten Wellenlängen zwischen der am Eingangsanschluß eingeführten Strahlung und der am Detektionsanschluß detektierten Strahlung.

**Revendications**

1. Dispositif spectroscopique à modulation de phase pour l'examen d'un tissu d'un sujet, les propriétés de dispersion et d'absorption de ce tissu étant déterminées par la migration des photons entre une borne d'entrée optique et une borne de détection optique appartenant à ce dispositif, celui-ci comprenant : un oscillateur adapté pour générer une onde porteuse de fréquence choisie compatible avec le retard de la migration photonique de cette borne d'entrée vers cette borne de détection ; une source lumineuse, connectée de manière opérationnelle à cet oscillateur, adaptée pour générer un rayonnement électromagnétique de longueur d'onde choisie c'est-à-dire dont l'intensité est modulée à ladite fréquence, ce rayonnement étant introduit dans le sujet par la borne d'entrée ; et un détecteur optique adapté pour détecter, au niveau de la borne de détection, le rayonnement ayant migré dans le tissu du sujet entre ces bornes ; ce dispositif étant caractérisé en ce qu'il comprend en outre ; un déphaseur multiple adapté pour produire, à partir du rayonnement introduit, des premier et second signaux de phase de référence dont les phases prédéfinies sont sensiblement différentes ; des premier et second mélangeurs symétriques doubles adaptés pour corréler les signaux de phase de référence et les signaux du rayonnement détecté de manière à produire ainsi respectivement un signal réel de sortie et un signal imaginaire de sortie ; et un moyen de traitement de signaux adapté pour déterminer, sur la base de ces signaux réel et imaginaire, une caractéristique choisie en relation avec les propriétés de dispersion et d'absorption dudit tissu.

2. Dispositif spectroscopique selon la revendication 1, caractérisé en ce qu'il comprend en outre : une source lumineuse, connectée de manière opérationnelle à l'oscillateur, adaptée pour générer un rayonnement électromagnétique d'une seconde longueur d'onde choisie dont l'intensité est modulée a ladite fréquence ; un coupleur adapté pour introduire dans le sujet, au niveau de la borne d'entrée, un rayonnement à chacune de ces longueurs d'onde : le détecteur optique étant en outre adapté pour détecter, au niveau de la borne de détection, le rayonnement de la seconde longueur d'onde ayant migré dans le tissu entre la borne d'entrée et celle de détection ; et un premier et un second commutateur électronique, fonctionnant tous les deux de manière synchrone avec le coupleur, conçus pour recevoir respectivement le signal réel de sortie et le signal imaginaire de sortie provenant des premier et second mélangeurs symétriques doubles, les commutateurs étant adaptés pour séparer respectivement les signaux réel et imaginaire en signaux pour chacune des longueurs d'onde.

3. Dispositif spectroscopique selon la revendication 2, caractérisé en ce qu'il comprend en outre : une source lumineuse, connectée de manière opérationnelle audit oscillateur, adaptée pour générer un rayonnement électromagnétique d'une troisième longueur d'onde choisie c'est-à-dire dont l'intensité est modulée à ladite fréquence ; le détecteur optique étant en outre adaptée pour détecter, au niveau de cette borne de détection, le rayonnement de cette troisième longueur d'onde ayant migré dans le tissu entre la borne d'entrée et celle de détection ; et les premier et second commutateurs électroniques, fonctionnant tous les deux de manière synchrone avec le coupleur, étant en outre conçus pour recevoir le signal réel de sortie et la signal imaginaire de sortie provenant respectivement des premier et second mélangeurs symétriques doubles et étant de plus adaptés pour séparer respectivement les signaux réels et les signaux imaginaires pour chacune des trois longueurs d'onde.

4. Dispositif spectroscopique selon l'une quelconque des revendications 1, 2, 3, caractérisé en outre en ce que ladite caractéristique choisie est une amplitude de signal, au moins pour une longueur d'onde choisie, cette amplitude étant définie comme étant la racine carrée de la somme des carrés du signal réel de sortie et du signal imaginaire de sortie pour cette longueur d'onde.

5. Dispositif spectroscopique selon l'une quelconque des revendications 2, 3 ou 4, caractérisé en ce qu'il comprend en outre : un détecteur à bande étroite adapté pour recevoir les signaux détectés par le détecteur optique et pour générer un signal de sortie en courant continu; un troisième commutateur électronique fonctionnant de manière synchrone avec le coupleur, conçu pour recevoir le signal de sortie en courant continu pour chacune des longueurs d'onde : et le moyen de traitement de signaux étant en outre adapté pour déterminer un indice de modulation, au moins pour une des longueurs d'onde, sous la forme du rapport des valeurs de l'amplitude du signal et de cette amplitude plus le signal de sortie en courant continu.

6. Dispositif spectroscopique selon l'une quelconque des revendications 1, 2 ou 3, caractérisé en outre en ce que la caractéristique choisie est le déphasage entre le rayonnement introduit au niveau de la borne d'entrée et celui détecté au niveau de la borne de détection, ce déphasage étant déterminé au moins pour une longueur d'onde choisie.

7. Dispositif spectroscopique selon la revendication 6, caractérisé en outre en ce que le moyen de traitement de signaux est en outre adapté pour déterminer le rapport des déphasages pour deux longueurs d'onde choisies.

8. Dispositif spectroscopique selon la revendication 7, caractérisé en outre en ce que le moyen de traitement de signaux est de plus adapté pour déterminer la saturation de l'hémoglobine sur la base de ce rapport des déphasages.

9. Dispositif spectroscopique selon l'une quelconque des revendications 1, 2 ou 3, caractérisé en outre en ce que la caractéristique choisie est le chemin de migration suivi par le rayonnement entre la borne d'entrée et celle de détection au moins pour une des longueurs d'onde choisies.

10. Dispositif spectroscopique selon l'une quelconque des revendications 1, 2 ou 3, caractérisé en outre en ce que l'oscillateur est de plus adapté pour fonctionner à une fréquent différente choisie de l'onde porteuse.

11. Dispositif spectroscopique selon la revendication 10, caractérisé en outre en ce que la caractéristique choisie du tissu est un déphasage entre le rayonnement introduit au niveau de la borne d'entrée et celui détecté au niveau de la borne de détection, ce déphasage étant déterminé au moins pour deux longueurs d'onde et au moins à deux fréquences choisies ; et en ce que le moyen de traitement de signaux est en outre adapté pour déterminer la saturation de l'hémoglobine sur la base de ces déphasages.

12. Dispositif spectroscopique selon l'une quelconque des revendications précédentes, caractérisé en outre en ce que les longueurs d'onde sont dans la gamme du visible et de l'infrarouge.

13. Procédé pour déterminer les propriétés de dispersion et d'absorption d'un tissu d'un sujet vivant, ces propriétés étant déterminées par la migration de photons entre une borne d'entrée optique et une borne de détection optique, ce procédé comprenant les étapes consistant à : générer une onde porteuse de longueur d'onde choisie compatible avec le retard de la migration photonique de la borne d'entrée vers la borne de détection introduire dans le sujet, au niveau de la borne d'entrée, un rayonnement électromagnétique de longueur d'onde choisie dont l'intensité a été modulée au niveau de cette onde porteuse ; détecter, au niveau de la borne de détection le rayonnement ayant migré dans ce tissu entre la borne d'entrée et celle de détection ; créer deux signaux de phase de référence dont les valeurs de phase prédéfinies sont sensiblement différentes ; comparer le rayonnement détecté aux deux signaux de référence et déterminer ainsi respectivement un signal réel de sortie et un signal imaginaire de sortie; et, sur la base de ces signaux réel et imaginaire, calculer une caractéristique choisie en relation avec les propriétés de dispersion et d'absorption du tissu.

14. Procédé selon la revendication 13, comprenant en outre les étapes consistant à : introduire dans le sujet au niveau de la borne d'entrée un rayonnement électromagnétique d'une seconde longueur d'onde choisie modulée au niveau de l'onde porteuse ; détecter, au niveau de la borne de détection, le rayonnement de cette seconde longueur d'onde ayant migré dans le tissu entre la borne d'entrée et celle de sortie ; comparer le rayonnement détecté pour la seconde longueur d'onde aux deux signaux de référence et déterminer ainsi respectivement pour cette seconde longueur d'onde un signal réel de sorte et un signal imaginaire de sortie; et sur la base de ces signaux réel et imaginaire pour chacune des longueurs d'onde choisies, calculer une caractéristique choisie en relation avec les propriétés de dispersion et d'absorption du tissu.

15. Procédé selon la revendication 14, comprenant en outre les étapes consistant à : introduire dans la sujet au niveau de la borne d'entrée un rayonnement électromagnétique d'une troisième longueur d'onde choisie modulée au niveau de l'onde porteuse ; détecter, au niveau de la borne de détection, le rayonnement de cette troisième longueur d'onde ayant migré dans le tissu entre la borne d'entrée et celle de détection ; comparer le rayonnement pour la troisième longueur d'onde aux deux signaux de référence et déterminer ainsi respectivement pour cette troisième longueur d'onde un signal réel de sortie et un signal imaginaire de sortie.

16. Procédé selon l'une quelconque des revendications 13, 14 et 15 répété à une seconde fréquence choisie, les propriétés de dispersion et d'absorption du tissu étant examinées en utilisant les caractéristiques choisies déterminées au moins pour deux longueurs d'onde et à deux fréquences.

17. Procédé selon l'une quelconque des revendications 14, 15 ou 16 dans lequel lesdites longueurs d'onde choisies sont dans la gamme du visible et de l'infrarouge.

18. Procédé selon l'une quelconque des revendications 13 à 17, dans lequel la caractéristique choisie est une amplitude de signal définie comme étant la racine carrée de la somme des carrés des signaux réel de sortie et imaginaire de sortie.

19. Procédé selon la revendication 18, dans lequel le procédé comprend en outre les étapes consistant à ; déterminer le signal en courant continu provenant du rayonnement détecté ; et calculer l'indice de modulation sur la base de ce signal en courant continu et de l'amplitude de signal.

20. Procédé selon l'une quelconque des revendications 14 à 17, dans lequel la caractéristique choisie est un déphasage entre le rayonnement introduit au niveau de la borne d'entrée et celui détecté au niveau de la borne de détection, ce déphasage étant déterminé au moins pour une des longueurs d'onde choisies.

21. Procédé selon la revendication 20, comprenant en outre les étapes consistant à ; déterminer le rapport des coefficients d'absorption du rayonnement pour chacune des longueurs d'onde choisies sur la base du rapport des déphasages correspondants.

22. Procédé pour déterminer la saturation de l'hémoglobine du tissu examiné ou pour fournir une valeur numérique utile dans la détermination d'une hémorragie localisée de ce tissu comprenant la définition du rapport des coefficients d'absorption par un procédé selon la revendication 21.

23. Procédé selon l'une quelconque des revendications 14 à 17, dans lequel la caractéristique choisie est le chemin suivi par la migration photonique entre le rayonnement introduit au niveau de la borne d'entrée et le rayonnement détecté au niveau de la borne de détection, ce chemin étant déterminé au moins pour une des longueurs d'onde choisies.

# FIG. 1

# FIG. 2

FIG. 3

$$A_{\lambda_1} = \sqrt{R^2_{\lambda_1} + I^2_{\lambda_1}}$$

$$A_{\lambda_2} = \sqrt{R^2_{\lambda_2} + I^2_{\lambda_2}}$$

$$M_{\lambda_1} = \frac{A_{\lambda_1}}{A_{\lambda_1} + DC_{\lambda_1}}$$

$$\frac{\theta_{\lambda_1}}{\theta_{\lambda_2}} = \frac{\tan^{-1}\frac{I_{\lambda_1}}{R_{\lambda_1}}}{\tan^{-1}\frac{I_{\lambda_2}}{R_{\lambda_2}}}$$